# EUROPEAN PATENT SPECIFICATION

(11) **EP 1 335 687 B1**
(45) Date of publication and mention of the grant of the patent: **10.01.2007**
(21) Application number: 01996398.2
(22) Date of filing: 15.11.2001
(51) Int. Cl.: A61F 2/44, A61K 31/717, A61K 31/715, A61K 31/765, A61P 19/00

(54) **METHOD FOR RESTORING A DAMAGED OR DEGENERATED INTERVERTEBRAL DISC**
VERFAHREN ZUR WIEDERHERSTELLUNG EINER GESCHÄDIGTEN BANDSCHEIBE
PROCEDE DE RESTAURATION D'UN DISQUE INTERVERTEBRAL ENDOMMAGE OU ATTEINT DE DEGENERESCENCE

(30) Priority: 15.11.2000 US 248226 P; 16.11.2000 US 248568 P
(43) Date of publication of application: 20.08.2003
(73) Proprietor: Bio Syntech Canada Inc., Laval, Québec H7V 4B3 (CA)
(72) Inventor: DESROSIERS, Eric André, Outremont, Québec H2V 1V7 (CA); CHENITE, Abdellatif, Kirkland, Québec H9H 4H6 (CA); BERRADA, Mohammed, Montréal, Québec H4J 2N2 (CA); CHAPUT, Cyril, Montréal, Québec H3R 2G1 (CA)
(74) Representative: Marks & Clerk Incorporating Edward Evans Barker
(86) International application number: PCT/CA2001/001623
(87) International publication number: WO 2002/040070

(56) References cited:
- WO-A-01/36000
- WO-A-01/41822
- WO-A-99/04720
- WO-A-99/07416
- CHENITE A ET AL: "Novel injectable neutral solutions of chitosan form biodegradable gels in situ" BIOMATERIALS, ELSEVIER SCIENCE PUBLISHERS BV., BARKING, GB, vol. 21, no. 21, 1 November 2000 (2000-11-01), pages 2155-2161, XP004216030 ISSN: 0142-9612

## Description

### BACKGROUND OF THE INVENTION

### (a) Field of the Invention

The invention relates to a minimally-invasive method for restoring a damaged or degenerated intervertebral disc using an injectable *in situ* setting formulation that is administered to the *pulposus nucleus* of the disc.

### (b) Description of Prior Art

Natural soft tissues, such as cornea, cartilage and intervertebral disc, are conveniently classified as hydrogel composites. About 70% of the population suffer or will suffer from back pains between the ages of 20-50. This weakness of our biped condition can be traced, in 80% of the cases, to faulty intervertebral discs. Those discs play the roles of a multidirectional articulation, and of a shock absorber. Their structure is complex. The outside shell of the disc, the ligamentous *annulus fibrosus,* is made of 10-20 concentric layers of overlapping collagen fibers, while its center is inflated with a semi-liquid cartilaginous substance, called the *nucleus pulposus,* exerting a strong colloid pressure. Above and below, the disc is limited by the hyaline cartilage end plates forming a porous junction between the disc and the adjacent vertebral bodies. The turgidity within that structure is mainly due to the proteoglycans of the nucleus, which contain fixed charges and are extremely hydrophilic. A quick compressive impact on the disc is transmitted directly to the annulus. However, if the load is maintained, water is expelled from the nucleus, through the end plates, to the vertebral bodies. As water is expelled, proteoglycan concentration increases within the disc and thereby the colloid pressure, until equilibrium is reached. The colloid pressure within the nucleus will then draw back the lost volume of fluid once the load is removed. Every day, the weight of our body compresses each intervertebral disc by about 10% of its height. That lost volume is regained during the night. The integrity of the proteoglycan pool of the nucleus is maintained through life by a few chondrocyte-like cells dispersed within the nucleus matter. Mechanical pumping action is essential for their nutrition and evacuation of metabolites since the discs are not vascularised.

With age, the concentration and composition of the proteoglycans within the nucleus changes, leading to a decrease in colloid pressure - and to the consequent decrease in disc height, by as much as 30%. It subjects the annulus to additional stress that can lead to delamination and hernia. Even without prior degeneration of the nucleus matter, a strong shock, or an unfortunate combination of compression and torsion will often lead to a hernia, where the integrity of the annulus is affected. The reduced height of a herniated disc does not allow the annulus to heal and often leads to painful irritation of the surrounding nerve roots. Conservative treatments include rest, heat, and pain management with non-steroidal anti-inflammatory drugs. Most of the cases will then heal, or become tolerated. However, for some (about 20%) of the cases, there is no other recourse than surgery: laminectomy, nerve root decompression, lumbar fusion, or even the installation of an artificial disc. In spite of the recent introduction of laparoscopic techniques and fusion cages, the surgical methods remain major -and expensive- interventions. Intervertebral fusion usually relieves pain, but loads the two adjacent discs with new, un-physiological stresses that often lead to repeat surgery within the next few years. The current artificial disc prosthesis is not a popular alternative, since they cannot, or hardly, meet the normal articular range of motion and fatigue resistance requirements.

In 1996, there were a total of 440,000 spinal surgical procedures performed worldwide (about 0.1% of the world population of 20-50 year olds). Of those, 40% involved spinal instrumentation (180,000 units/procedures and $368 million US) with a total cost for each typical spinal instrumentation surgery at $45,000US. This procedure is gradually being replaced by laparoscopic implantation of fusion cage, at the lower cost of $ 9,000 US, and with faster post-surgical recovery. By 2001, it is anticipated that at least 45% of the interventions will be fusion cage lap surgeries. An efficient non-surgical procedure would cost a fraction of the surgery cost and have a broader appeal to 'back sufferers' (those who would normally go through surgery and those who endure the pain to avoid surgery).

A great number of treatment methods and materials for repairing or replacing intervertebral discs have been proposed.

Two developmental approaches exist to surgically treat intervertebral discs: the first one focuses on designing artificial total discs, the other targets artificial nucleus.

The artificial total disc is developed to replace the complete disc structures: *fibrosus annulus, nucleus pulposus* and endplates. Artificial discs are challenged by both biological and biomechanical considerations, and often require complex prosthesis designs. Metals, ceramics and polymers have been incorporated in various multiple component constructions. Metal and nonmetal disc prostheses have been proposed, including a metallic or ceramic porous disc body filled with a poly(vinyl alcohol) hydrogel (US5,314,478). Elastic polymers, elastomers and rubbers have been also proposed for designing artificial disc implants. An alloplastic disc was presented again, consisting in a hollow elastomer, preferably a vulcanizable silicone such as Silastic®, that is shaped to mimic the intervertebral disc to be replaced (L. Daniel Eaton, US6,283,998 B1). Biedermann *et al.* (US6,176,882 B1) recently proposed a complex geometrical concept of artificial intervertebral disc, consisting in two side walls, a front wall and a back wall, all walls being disposed specifically one in regard to the other.

In the most recent years, the artificial nucleus takes advantage over the artificial total disc. Its main advantage is the preservation of disc tissues, the annulus and the endplates. Artificial nucleus also enable to maintain the biological functions of the preserved natural tissues. Furthermore the replacement of the nucleus is surgically less complicated and at risk than the total replacement of the intervertebral disc. One limitation of the artificial nucleus resides in the need of relatively intact annulus and endplates, which means the nucleus replacement must be performed when disc degeneration is at an early stage. Finally, the nucleus surgery is less at risk for the surrounding nerves, and if the replacement with an artificial nucleus failed clinically, it remains the possibility to convert to a fusion or a total disc replacement.

Artificial materials for nucleus replacement have been selected among metals such as stainless-steel balls, and more now among nonmetals such as elastomers, and polymeric hydrogels. The physiological *nucleus pulposus* is often reported as being close to a natural collagen-glycosaminoglycans hydrogel, with a water content about 70-90% (wt.). In comparison to the nucleus, polymeric hydrogels as well as pure natural hydrogels may present closed material properties. Those artificial hydrogels have been enclosed within outer envelopes of various shapes (tubes or cylinders...) and composition (polyethylene, polyglycolide...). The polymers introduced in artificial disc devices comprise polyethylene, poly(vinyl alcohol), polyglycolide, polyurethane, and the like.

In last years, artificial nucleus materials have been proposed. Bao and Higham (US5,192,326) described a prosthetic nucleus, formed of multiple hydrogel beads, having a water content of at least 30%, entrapped within a closed semi-permeable membrane. The porous membrane retained the beads but allowed the fluids to flow in and out.

Krapiva (US5,645,597) proposed to remove the nucleus from the disc, to insert an elastic flexible ring, an upper membrane and a lower membrane within the space, and to fill the inner chamber with a gel-like substance. The RayMedica Inc. medical device company proposed an elongated pillow-shaped prosthetic disc nucleus, composed basically of a outer soft jacket filled with a hydrogel (Ray *et al.,* US5,674,295). In a very similar way, Ray and Assel (US6,132,465) also disclosed a more constraining jacket filled again with a hydrogel..

Lawson (US6,146,422) proposed a prosthetic nucleus device, in a solid form, having an ellipsoidal shape and generally made of polyethylene.

A swellable biomimetic and plastic composition, with a hydrophobic phase and a hydrophilic phase, was used by Stoy (US6,264,695B1), including a xerogel (a gel formed in a nonaqueous liquid). Liquids may be selected among water, dimethyl sulfoxide, glycerol, and glycerol monoacetate, diacetate or, formal, while hydrophilic phases consisted in nitrile containing, carboxyl, hydroxyl, carboxylate, amidine or amide chemicals.

Bao and Higham (US6,280,475B1) described a hydrogel prosthetic nucleus to be inserted within the intervertebral disc chamber. Solid hydrogels prepared by freeze-thawing poly(vinyl alcohol) in water/dimethyl sulfoxide solutions comprise 30 to 90% of water, and have typically compressive strengths about 4 MNmm⁻². Finally, Ross et al. (US6,264,659B1) also eliminated the remaining nucleus of a ruptured annulus, and injected a thermo-plastic material that was preheated at a temperature over 50°C. This thermoplastic material became less flowable when returned at a temperature near 37°C. *Gutta percha* is the only described thermoplastic material.

An intervertebral disc nucleus prosthesis was again described by Wardlaw (WO99/02108), consisting in a permeable layer of an immunologically neutral material where a hydrogel was injected. Poly(vinyl alcohol) was given as an example of hydrogel. More recently, a combination of polymeric hydrogels was prepared typically from poly(vinyl alcohol) and poly(vinyl pyrollidone) or its copolymers, and applied to the replacement of the disc nucleus (Marcolongo and Lowman, WO01/32100A2).

Other nucleus replacement techniques were disclosed where a polyurethane was polymerized *in situ* within a inflatable bag inserted in the *annulus fibrosus.*

Most recently, living biologicals were combined with artificial materials to be used as regeneration or replacement devices for the nucleus. Chin Chin Gan, Ducheyne et al. (US6,240,926B1) used hybrid materials consisting generally in intervertebral disc cells, isolated from the disc tissues, adhered and cultured onto artificial biomaterials. Typical supporting biomaterials may be selected among polymeric substrata, such as biodegradable polylactide, polyglycolide or polyglactin foam, and porous inorganic substrata, such as bioactive glass or minerals. The supporting substrata were generally microparticles (beads, spheres...) or granules, about 1.0 mm in size or less.

In a same way, Stoval (WO99/04720) proposed a method for treating herniated intervertebral discs, where fibroblasts, chondrocytes or osteoblasts were incorporated within a hydrogel. The cell-containing suspension was adhered onto one surface of the *annulus fibrosus,* or was injected as a cell-containing suspension into the herniated disc to form a cell-containing hydrogel. Chondrocytes isolated from the intervertebral disc were preferably used to develop this cell-containing composition.

Chenite et al. in *Biomaterials,* 21, 2155-2161, 2000, disclosed a temperature dependent gel composition having a physiological pH which can be prepared in liquid below room temperature and which turns into a gel upon heating at a higher temperature.

International Publication WO 99/07416 relates to a temperature controlled pH-dependent formation of ionic polysaccharide gels having a specific formula. The gels described therein are composed of chitosan and a salt of polyol or sugar. When these two components are mixed together at a low temperature, they form a liquid but viscous composition which, upon heating, turns to the gel.

International Publication WO 99/04720 describes a method for using a cell containing a hydrogel suspension for treating a herniated intervertebral disc by implanting the cell suspension into a patient. Although this application solves the problem of material compatibility, it still requires surgical intervention to implant the gel in the disc, as the composition is a hydrogel and thus has the viscosity and structure of a hydrogel which is not a liquid.

Degeneration of the *nucleus pulposus* of the intervertebral disc is one primary step of most intervertebral disc problems and low back pain. The nucleus is a hydrogef-like biological material with a water content above 70%, and generally around 90%. A water content decrease (water loss) is the first reason for the disc degeneration. This water loss may significantly reduce the ability of the disc to withstand mechanical stresses, thus reducing the biomechanical performances of the inter-vertebral discs. Further steps of disc degeneration and damage include disc protrusion, where the nucleus substance still remains within the annulus, then disc rupture or prolapse, where the nucleus substance flows from the annulus. Ruptures of the intervertebral disc may result in spasms, compressed sort-tissues, nerve compression and neurological problems. Disc compression with no major annulus ruptures is the primary stage of the disc problems, and is often caused by ongoing nucleus degeneration and function loss.

Isolated and early treatments by applying non- or minimally-invasive methods focused only on the degenerated or damaged tissues should be envisaged and preferred, It is clear that early treatments of degenerated or less operational *nucleus pulposus* would restore the cushioning, mechanical support and motion functions to the disc and spine.

It would be highly desirable to be provided with a novel minimally-invasive method for restoring damaged or degenerated intervertebral discs.

It would be more desirable to be provided with a novel minimally-invasive method for obtaining restoration of disc functions at an early stage, particularly before any advanced degeneration or damages resulting into disc rupture and fragmentation.

It would be still more desirable to be provided with a novel minimally-invasive method for restoring the functions of the *pulposus nucleus* of the disc, before disc compression becomes more painful and disabling.

### SUMMARY OF THE INVENTION

One object of the present invention is to provide a new minimally-invasive method and formulations for restoring a damaged or degenerated intervertebral disc.

In accordance with the present invention there is provided the use of a nucleus pulposus formulation comprising:
a) 0.1 to 5.0% by weight of a water-soluble polymer selected from the group consisting of cellulosic, polysaccharide and polypeptidic, or a mixture thereof, and
b) 1.0 to 20% by weight of a water-soluble salt selected from the group consisting of phosphate, glycerol-phosphate, glucose-phosphate, fructose phosphate, carbonate, sulfate, sulfonate and a salt of polyol or sugar selected from the group consisting of mono-phosphate dibasic salt, mono-sulfate salt and a mono-carboxylic acid salt;
wherein said formulation has a pH ranging from 6.5 to 7.4, and turns into a gel within a temperature range from 20 to 70°C, said gel having a physiologically acceptable consistency for increasing the thickness of the disc, providing a mechanical support once injected in the disc, in the manufacture of a medicament for restoring a damaged or degenerated intervertebral disk.

Also provided according to the present invention is the use of a nucleus pulposus formulation comprising:
a) 0.1 to 5.0% by weight of chitosan or collagen or a derivative thereof, or a mixture thereof; and
b)
   i) 1.0 to 20% by weight of a salt of polyol or sugar selected from the group consisting of mono-phosphate dibasic salt, mono sulfate salt and a mono-carboxylic acid salt of polyol or sugar; or
   ii) 1.0 to 20% by weight of a salt selected from the group consisting of phosphate, carbonate, sulfate, and sulfonate, or the like; and
c) 0.01 to 10% by weight of a water-soluble chemically reactive organic compound;
wherein said formulation has a pH ranging from 6.5 to 7.4, and turns into a gel within a temperature range from 4 to 70 C, said gel having a physiologically acceptable consistency for increasing the thickness of the disc, providing a mechanical support once injected in the disc, in the manufacture of a medicament for restoring a damaged or degraded invertebral disc.

The formulation may contain chondroitin sulfate, hyaluronic acid, poly(ethylene glycol), or a derivative thereof, or a bioactive agent, a drug, such as a cell stimulant like for example growth factors and cytokines.

The injectable formulation Is either viscous or form a solid or gel *in situ.*

The salt can be a mono-phosphate dibasic salt selected from the group consisting of glycerol, comprising glycerol-2-phosphate, sn-glycerol 3-phosphate and L-glycerol-3-phosphate salts, or a mono-phosphate dibasic salt and said polyol can be selected from the group consisting of histidinol, acetol, diethylstilbestrol, indole-glycerol, sorbitol, ribitol, xylitol, arabinitol, erythritol, inositol, mannitol, glucitol and a mixture thereof. The mono-phosphate dibasic salt and said sugar are preferably selected from the group consisting of fructose, galactose, ribose, glucose, xylose, rhamnulose, sorbose, erythrulose, deoxy-ribose, ketose, mannose, arabinose, fuculose, fructopyranose, ketoglucose, sedoheptulose, trehalose, tagatose, sucrose, allose, threose, xylulose, hexose, methylthioribose, methylthio-deoxy-ribulose, and a mixture thereof, or is selected from the group consisting of palmitoyl-glycerol, linoleoyl-glycerol, oleoyl-glycerol, arachidonoyl-glycerol, and a mixture thereof. Alternatively, the injectable solution can be selected from the group consisting of chitosari-β-glycerophosphate, chitosan-α-glycerophosphate, chitosan-glucose-1-glycerophosphate, chitosan-fructose-6-glycerophosphate, and methyloellulose-phosphate.

The injectable formulation, can also comprise a biocompatible physiologically acceptable polymer.

The injectable formulation preferably comprises a polymer that is polymerized or cross-linked after being injected *in situ.*

The injectable formulation may comprise at least one saturated or unsaturated fatty acid selected from the group consisting of palmitate, stearate, myristate, palmitoleate, oleate, vaccenate and linoleate. It may be a mixture of several fatty acids. The fatty acid may be mixed with a metabolically absorbable solvent or liquid vehicle to reduce viscosity and allow injectability.

### BRIEF DESCRIPTION OF THE DRAWINGS

Figs. 1A illustrates the intervertebral disc as anatomically disposed between vertebra within the spine (as shown by the black arrow);
Fig. 1B is a cross-sectional view along line A-A of Fig. 1A;
Figs. 2A to 2E illustrate the different stages of the intervertebral damages: the normal disc (Fig. 2A), the compressed disc (Fig. 2E), the disc protrusion (Fig. 2B), and the disc rupture (Figs. 2Cand 2D);
Figs. 3A to 3D illustrate a method of percutaneously administering an injectable *in situ* setting formulation, which will set *in situ* to form a highly viscous solution, a gel or a solid, to the nucleus pulposus of the intervertebral disc;
Fig. 4 illustrates the intervertebral disc after injection with a red colored dyed gel in accordance with the present invention.
Figs. 5A and 5B illustrates an example of a radiography before (Fig. 5A) and after (Fig. 5B) disc injection;
Figs. 6A to 6C illustrate the *in vitro* cytotoxicity of mPEG2000 (Fig. 6A), B.NHS (Fig. 6B) and MPEGA.5000 (Fig. 6C) used to design *in situ* setting (gelling) formulations; and
Figs. 7A and 7B illustrate the tissue reaction toward *in situ* setting formulations of the present invention, using Chitosan-mPEG-NHS in Fig. 7A and Chitosan in Fig. 7B, injected subcutaneously in rats [Saffranin-O/Fast Green (magnification x40] sacrificed at 21 days post-injection.

### DETAILED DESCRIPTION OF THE INVENTION

In accordance with the present invention, an injection of a thermogelling chitosan-based formulation into a damaged or degenerated disc allows to restore its volume and thickness thereby restoring the damaged or degenerated disc. The method of the present invention affords to the patient one last non-surgical option that solves the problem. Indeed, for indications where the nucleus has not extruded through the annulus, the gel solution can be injected within the disc using a syringe, in a procedure similar to a common diagnostic discography, to gel *in situ.* The gel solution, once injected and prior to gelling, mixes with the remaining cells and nucleus matter to form an elastic hydrogel *in situ* upon gelation. The gel so obtained supports the physiological load through intrinsic elasticity and colloid pressure, while allowing the normal pumping action. Furthermore, the structural integrity of this gel limits hernia damage by preventing extrusion of the nucleus mater through annulus defects.

### A Novel Method and Formulation

In the development of the present invention, it was found that the thickness of intervertebral discs could be restored by the injection of an appropriate formulation. An appropriate formulation first needs to be liquid enough to be injectable. After injection, the mechanical properties of such a formulation become compatible with the biomechanical function of the discs, by gelling or becoming highly viscous. Finally, the injected product has to be non-toxic, biocompatible, and to have an extended residence time in the discs to provide a durable restoration of the discs.

A preferred formulation for carrying out the method is a thermogelling chitosan-based aqueous solution. The thermogelling chitosan-based solution is easily injectable, turns into a gel *in situ* and provides substantial mechanical support to the surrounding soft tissues. The solution remains liquid below body temperature and gels after injection as it is warmed to body temperature.

However, other solutions as described in the summary of the invention are also suitable to be used in the present invention.

With the method of the present invention, the gel so-obtained once injected is chondrogenic, and supports chondrocyte growth and extracellular matrix deposition. The restoration of the disc's thickness, combined with the introduction of a chondrogenic matrix supports the load, relieve the pain and promote the healing and regeneration of a healthy disc.

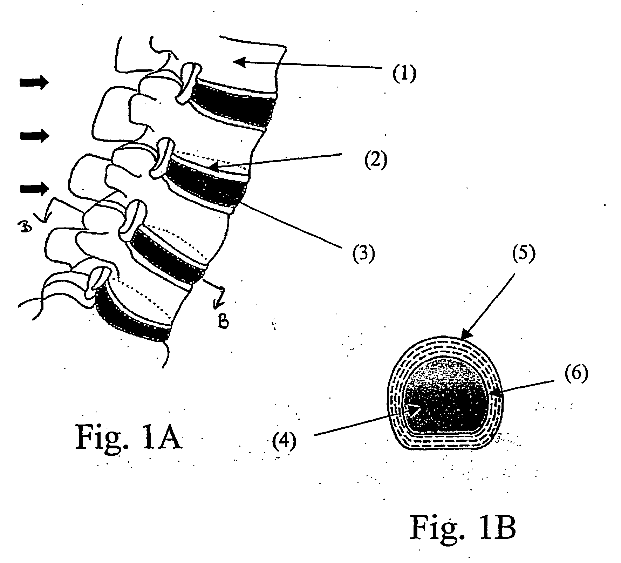

In one embodiment of this invention, the method uses an injectable *in situ* setting formulation to be administered percutaneously to the *nucleus pulposus* of the intervertebral disc. This enables to increase and restore the thickness and volume of the intervertebral disc as well as its cushioning and mechanical support effects. The anatomy of an spine with the intervertebral disk is illustrated in Figs. 1A and 1B. Fig .1A illustrates the intervertebral disc (3) [anullus fibrosus and nucleus pulposus] and endplates (2) as anatomically disposed between vertebra (1) within the spine shown by the black arrow. The intervertebral disc (3) is composed of radial fibrous sheets (6) loosely bonded together, each alternative sheet consisting of tough fibers oriented oppositely, a outer annulus membrane (5), a inner annulus membrane (6) (all three composing the Anullus fibrosus), and the *nucleus pulposus* (4).

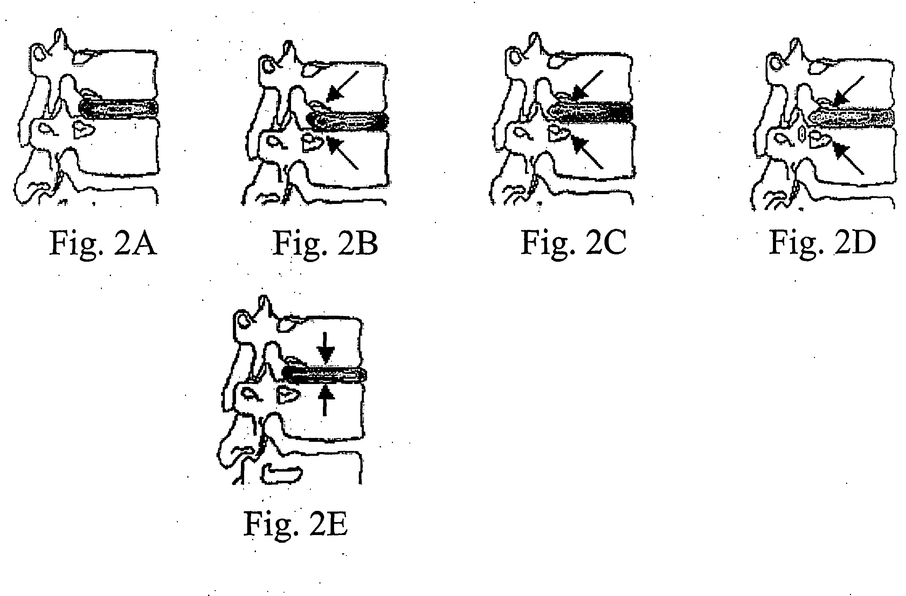

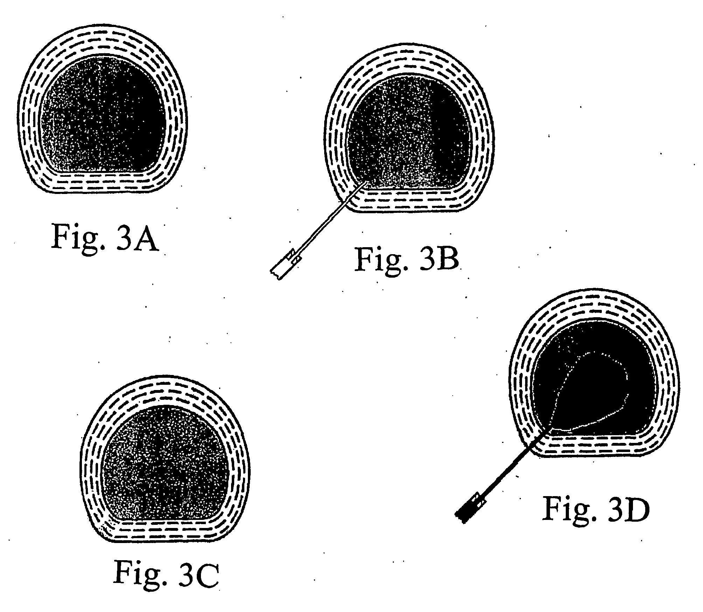

Figs. 2A to 2E illustrate different stages of the intervertebral disc damages. Disc protusion (Fig. 2B) includes contained disc where disc is herniated, goes out of its normal location (to the spinal canal), but is not ruptured. Disc rupture (Fig. 3C) may lead to sequestered disc, with sequestered fragments of disc diffusing.

The term "formulation" refers herein to any composition, including solution and dispersion that is prepared for the described method. The term *"**in situ* setting" refers herein to the property of having some formulation properties changed once injected into the intervertebral disc. *"In situ* setting" includes any setting that is time-delayed or stimulated *in vivo* by physiological parameters such as the temperature, pH, ionic strength, etc. *"In situ* setting" typically comprises viscosity-increasing, (self-) gelling, thermo-gelling, (self-) polymerizing, cross-linking, hardening, or solid-forming. Here, it is generally used to describe a reaction or formulation change associated to a gelling, polymerizing or crosslinking that occurs *in situ* within the intervertebral disc. This means that the formulation, flowable and injectable at the time of administration, will gel, crosslink or polymerize to form a gel-like or solid material *in situ.*

The described method may be associated with other surgical techniques, minimally invasive, such as the cleaning of the *nucleus pulposus* (aspiration), a biochemical digestion of the *nucleus pulposus* or a preliminary re-inflating of the intervertebral disc (balloon).

In the preferred embodiments of this invention, the injectable *in situ* setting formulation is aqueous (contains water), and turns into a gel *in situ* preferably by the action of temperature (thermogelling). The formulation is then said thermogelling. It is preferably thermogelling, gelling by a temperature change, and preferably by increasing the temperature from a temperature below the body temperature to the body temperature (near 37°C).

In the preferred embodiments of this invention, the injectable *in situ* setting formulation is aqueous (contains water), and turns into a gel *in situ* through a covalent chemical reaction (crosslinking or polymerizing). The formulation is then said crosslinked or polymerized.

In the preferred embodiments of this invention, the injectable *in situ* setting formulation preferably comprises an aqueous solution containing a biopolymer such as a cellulosic, a polypeptidic or a polysaccharide or a mixture thereof. It may consist in a biopolymer solubilized in an aqueous medium. One preferred biopolymer is chitosan, a natural partially N-deacetylated poly(N-acetyl-D-glucosamine) derived from marine chitin. Other preferred biopolymers include collagen (of various types and origins). Other biopolymers of interest include methyl cellulose, hydroxyethyl cellulose, hydroxypropyl methyl cellulose, and the like.

In the preferred embodiments of this invention, the injectable *in situ* setting formulation preferably comprises an aqueous solution containing a water-soluble dibasic phosphate salt. It may contain a mixture of different water-soluble dibasic phosphate salts. The preferred dibasic phosphate salts comprise dibasic sodium and magnesium mono-phosphate salts as well as monophosphate salt of a polyol or sugar. This does not exclude the use of water-soluble dibasic salts other then phosphate, such as carboxylate, sulfate, sulfonate, and the like. Other preferred formulations of the method may contain hyaluronic acid or chondroitin sulfate or synthetic polymers such poly(ethylene glycol) or poly(propylene glycol), and the like.

In the preferred embodiments of this invention, there is provided a method for restoring a damaged or degenerated intervertebral disc, said method comprising the step of injecting an injectable formulation, such as a thermogelling chitosan-based aqueous solution, into the *nucleus pulposus* of the damaged or degenerated disc of a patient, said solution once injected combines with nucleus matters and host cells, and becomes viscous, pasty or turns into a gel *in situ* in the disc for increasing the thickness of the damaged or degenerated disc, said solution being retained within the *annulus fibrosus* for providing restoration of the damaged or degenerated disc. Figs. 3A to 3D illustrate a method of percutaneously administering an injectable *in situ* setting formulation to the *nucleus pulposus* of the intervertebral disc. Fig. 3A illustrates a compressed disc (*Annulus fibrosus* + *Nucleus pulposus*), whereas Fig. 3B illustrates an injection via a needle performed through the *annulus fibrosus* sheets to the *nucleus pulposus. Fig. 3C illustrates that the in situ* setting formulation is injected into the nucleus pulposus and mixed with the nucleus matter. Fig. 3D shows that a homogeneous mixing is reached *in situ,* and the final setting takes place within the disc.

In other embodiments, the injectable formulation is a thermogelling solution which comprises 0.1 to 5.0% by weight of a water-soluble cellulosic or polysaccharide or polypeptide or a derivative thereof, or any mixture thereof; and 1.0 to 20% by weight of a salt of polyol or sugar selected from the group consisting of mono-phosphate dibasic salt, mono-sulfate salt and a mono-carboxylic acid salt of polyol or sugar, or 1.0 to 20% by weight of a salt selected from the group comprising phosphate, carbonate, sulfate, sulfonate, and the like; wherein the solution has a pH ranging between 6.5 and 7.4, is stable at low temperatures such as below 20°C, and turns into a gel within a temperature range from 20 to 70°C. The gel has a physiologically acceptable consistency for increasing the thickness of the disc, providing a mechanical support once injected in the disc. The preferred polysaccharide or polypeptide is chitosan or collagen.

In other embodiments, the injectable formulation is a thermogelling solution which comprises 0.1 to 5.0% by weight of a water-soluble cellulosic or polysaccharide or polypeptide or a derivative thereof, or any mixture thereof; and 1.0 to 20% by weight of a salt of polyol or sugar selected from the group consisting of mono-phosphate dibasic salt, mono-sulfate salt and a mono-carboxylic acid salt of polyol or sugar, or 1.0 to 20% by weight of a salt selected from the group comprising phosphate, carbonate, sulfate, sulfonate, and the like; and a 0.01 to 10% by weight of a water-soluble reactive organic compounds; wherein the solution has a pH ranging between 6.5 and 7.4, and turns into a gel within a temperature range from 4 to 70°C. The gel has a physiologically acceptable consistency for increasing the thickness of the disc, providing a mechanical support once injected in the disc. The preferred polysaccharide or polypeptide is chitosan or collagen.

The water-soluble chemically reactive organic compounds comprise typically water-soluble molecules that are mono- or di-functionalized with chemical groups reactive with amine groups (-NH₂). Examples include poly(ethylene glycol) di-glycidyl ether, poly(ethylene glycol) di-tresylate, poly(ethylene glycol) di-isocyanate, poly(ethylene glycol) di-succinimidyl succinate, poly(ethylene glycol) di-succinimidyl propionate, di-succinimidylester of carboxymethylated poly(ethylene glycol), poly(ethylene glycol) di-benzotriazole carbone, carbonyldimidazole di-functionalized poly(ethylene glycol), or poly(ethylene glycol) dinitrophenyl carbonate, but also methoxyPEG-succinoyl-N-hydroxysuccinimide ester (mPEG-suc-NHS), methoxyPEG-carboxy=-methyl-NHS (mPEG-cm-NHS), and the like. "Chemically reactive" refers herein to any molecules or compounds that bring chemical groups susceptible to react covalently toward other specific chemical groups.

The salt can be a mono-phosphate dibasic salt selected from the group consisting of glycerol, comprising glycerol-2-phosphate, sn-glycerol 3-phosphate and L-glycerol-3-phosphate salts, or a mono-phosphate dibasic salt and said polyol is selected from the group consisting of histidinol, acetol, diethylstilbestrol, indole-glycerol, sorbitol, ribitol, xylitol, arabinitol, erythritol, inositol, mannitol, glucitol and a mixture thereof. The mono-phosphate dibasic salt and said sugar are preferably selected from the group consisting of fructose, galactose, ribose, glucose, xylose, rhamnulose, sorbose, erythrulose, deoxy-ribose, ketose, mannose, arabinose, fuculose, fructopyranose, ketoglucose, sedoheptulose, trehalose, tagatose, sucrose, allose, threose, xylulose, hexose, methylthioribose, methylthio-deoxy-ribulose, and a mixture thereof, or is selected from the group consisting of palmitoyl-glycerol, linoleoyl-glycerol, oleoyl-glycerol, arachidonoyl-glycerol, and a mixture thereof.

Alternatively, the injectable formulation can comprise aqueous solutions be selected from the group consisting of chitosan-β-glycerophosphate, chitosan-α-glycerophosphate, chitosan-glucose-1-glycerophosphate, and chitosan-fructose-6-glycerophosphate.

Among the aqueous formulations, having possible thermogelling capacities, of interest for the present invention, we may select chitosan-β-glycerophosphate, chitosan-α-glycerophosphate, chitosan-glucose-1-glycero-phosphate, chitosan-fructose-6-glycerophosphate, but equally collagen-β-glycerophosphate, methyl cellulose-sodium phosphate, hydroyethyl cellulose-sodium phosphate, etc.

In other embodiments of this invention, the injectable *in situ* setting formulation is nonaqueous (does not contain water) and solid or gel forming (turns into a solid or gel *in situ*).

In other embodiments of this invention, the injectable *in situ* setting formulation is nonaqueous (does not contains water), and turns into a solid *in situ* by the action of temperature (thermosetting). The formulation is said thermosetting.

In another embodiment of this invention, the injectable *in situ* setting formulation is nonaqueous and comprises an organic solvent or a mixture of organic solvents. Metabolically absorbable solvents are preferably selected (triacetin, ethyl acetate, ethyl laurate, etc). "Metabolically absorbable" refers herein to any chemicals or materials that are a) safely accepted within the body with no adverse reactions, and b) completely eliminated from the body over time through natural pathways or internal consumption. "Metabolically acceptable" refers to any chemicals or materials that are safely accepted within the body with no adverse reactions or harmful effects.

In another embodiment of this invention, the injectable *in situ* setting formulation is nonaqueous and contains at least one fatty acid or a mixture of fatty acids. The injectable formulation comprises saturated or unsaturated fatty acid selected from the group consisting of palmitate, stearate, myristate, palmitoleate, oleate, vaccenate and linoleate. It may be a mixture of several of these fatty acids. The fatty acid may be mixed with a metabolically absorbable solvent or liquid vehicle to reduce viscosity and allow injectability.

In other embodiments of the invention, a bioactive agent or drug is incorporated to the injectable *in situ* setting formulation. The bioactive agent or drug may be a peptide, a protein, a synthetic drug, a mineral, and the like. It is preferably a cell stimulant selected in a group comprising growth factors and cytokines. It may be also a healing enhancer, a pain relief agent, anti-inflammation agent.

In other embodiments of the invention, a nonsoluble solid component is incorporated to the injectable *in situ* setting formulation. It may be a solid particulate, e.g. microparticles, microbeads, microspheres or granules, of organic or inorganic composition.

In the present invention, the injectable *in situ* setting formulation is administered percutaneously to the intervertebral disc, in a minimally invasive way, to the *nucleus pulposus.* At the time of administration, the formulation has a viscosity that enables an easy and convenient minimally-invasive administration. At this step, the formulation is flowable, injectable, and typically has a viscosity above 10 mPa.s. It is intended that the formulation viscosity at the time of injection can be adjusted accordingly by acting onto the composition of the formulation, or by applying the appropriate shearing stress onto the formulation.

### Intended Use of the Formulation

Spine diseases can occur on many levels. In ageing adults, common back problems involve disc problems or nerve dysfunction leading to leg pain, numbness, tingling, weakness, back pain, unsteadiness and fatigue, etc. Nerve dysfunction at the level of the spine may lead to severe disabling pain and paralysis.

Nerve compression or spinal stenosis generally involves the disc, facet joints and ligaments (*ligamentum flavum,* posterior longitudinal ligament). The surgical treatment for patients suffering from nerve compression must be adapted to the situation. Common surgical procedures include discectomy (herniated disc), laminotomy (to open up more space posteriorly in the spinal canal), laminectomy (to unroof the spinal canal posteriorly), and foramenotomy (to open up the neuroforamen). These techniques may also be used in combination to ensure a proper decompression of the nerve elements.

Percutaneous decompression of intervertebral discs is performed currently, with more than 500,000 procedures during the past twenty years. Enzymatic digestion of the disc core with chymopapain, suction/cutting technique (Nucleotorny), and laser-induced tissue vaporization are the common techniques used for disc decompression. They give good to excellent results when applied to properly selected patients, but also present some serious disadvantages. Enzymatic treatment was associated with disc collapse and instability, and was also associated with cases of paralysis secondary to nerve damage. Chemopapain treatments may be also responsible for serious allergic reactions. The suction/cutting method (Nucleotomy) may be difficult to place correctly and seems to be often uncomfortable for the patient. Laser techniques can be associated with high levels of heat generation at the nerves and disc, as well as post procedure pain and spasm.

In the present invention, an early-stage method is proposed to augment a degenerated *nucleus pulposus* of an intervertebral disc. The method may be associated to additional treatments of the intervertebral disc, such as the partial removal or (biochemical) digestion of nucleus materials or the inflating of the disc. Inflation of the intervertebral disc may be performed by inserting a needle to the nucleus through the annulus, by inserting a balloon and inflating it *in situ,* then by filling the inflated disc with the formulation. It may also be associated with nucleoplasty, a percutaneous diskectomy performed through a small needle introduced into the posterior disc. A multifunctional device enables to ablate or remove tissue, while alternating with thermal energy for coagulation. This technique is used for herniated disc decompression.

In the proposed method, a low viscosity formulation, self-setting *in situ,* is injected into an unruptured, closed *annulus fibrosus.* It is mixable with the nucleus chemical and biological materials, and form rapidly a gel or solid *in situ.* The formulation is injected easily, with a minimal pressure, through the fine tube of a needle, trocar or catheter. Typical tube gauge ranges from 13 to 27. The length of the fine tube is adapted to endoscopic or laparascopic instruments as well as any methods for percutaneous administration. Injections are performed by instruments or devices that provide an appropriate positive pressure, e.g. hand-pressure, mechanical pressure, injection gun, etc. One representative technique is to use a hypodermic syringe.

The formulation is administered by injection through the wall of intact *annulus fibrosus* into the *nucleus pulposus.* It is preferable for the proposed method that the *annulus fibrosus* is intact at least at 90%.

The advantage of the present method is that the entire intervertebral disc is not removed to treat the degenerated disc. The *nucleus pulposus* may be eventually the only tissue to be removed. In the degenerated disc, the *nucleus pulposus* is the tissue that presents a decrease of the mechanical performances, or has partly or totally disappeared.

The present method of the invention will be more readily understood by referring to the following examples, giving some examples of *in situ* setting formulations that can be used. These examples are given to illustrate the invention rather than to limit its scope, and are not exclusive of any other formulations and methods that prove to be appropriate in regard to the presented invention.

### EXAMPLE I

### Effect of composition on pH of solution and occurrence of gelation

A mother acidic solution made of a Water/Acetic acid was prepared for all experiments. The pH of this mother acidic solution was adjusted to 4.0. High molecular weight (M.w. 2,000,000) Chitosan powder was added and dissolved in a volume of the mother acidic solution so as to produce Chitosan solutions having Chitosan proportions ranging from 0.5 to 2.0% w/v (Table 1). Table 1 reports the measured pH for the different samples.

**Table 1**

| **Chitosan Aqueous Solutions and pH levels** | | | | |
|---|---|---|---|---|
| Chitosan conc. (w/v) | 0.5 | 1.0 | 1.5 | 2.0 |
| pH of Chitosan Sol. | 4.68 | 4.73 | 5.14 | 5.61 |

Glycerophosphate was added to the chitosan solutions and induces a pH increase. Table 2 shows the effect of glycerophosphate concentration on different chitosan solution. The concentration of glycerophosphate ranges from 0.065 to 0.300 mol/L. The chitosan/glycerophosphate solutions in glass vials were maintained at 60 and 37°C, and bulk and uniform gelation was noted within 30 minutes at 60°C and 6 hours at 37°C (Table 2). Chitosan and beta-glycerophosphate components individually influence the pH increase within the aqueous solutions, and consequently influence the Sol to Gel transition. As well as the dissolved materials, the initial pH of the mother water/acetic acid solution would also influence the Sol to Gel transition, but this potential effect seems to be limited by the counter-action of the chitosan solubility, which depends on the pH of the solution.

**Table 2**

| **Gelation of Chitosan/Glycerophosphate Compositions** | | | | | | |
|---|---|---|---|---|---|---|
| Chitosan conc. (w/v) | 1.5 | | | 2.0 | | |
| pH of Chitosan Sol. | 5.14 | | | 5.61 | | |
| GP conc. (mol/L) | 0.130 | 0.196 | 0.260 | 0.130 | 0..196 | 0.260 |
| pH of Chitosan-GP Sol. | 6.64 | 6.83 | 6.89 | 6.78 | 6.97 | 7.05 |
| Gelation 60°C | < 30 min. | < 30 min. | < 30 min. | < 30 min. | < 30 min. | < 30 min. |
| 37°C | No | No | No | No | < 6 hrs | < 6 hrs |

### EXAMPLE II

### Crosslinkable Chitosan Gel compositions as delayed self-setting systems

Homogeneous Chitosan Gels Cross-Linked with Glyoxal was prepared as delayed gelling systems: 0.47 g of chitosan (85% deacetylated) was entirely dissolved in 20 mL of HCl solution (0.1 M). The chitosan solution so obtained had a pH of 5. This solution was cooled down to 4°C and added with ∼ 0.67 g of glycerol-phosphate disodium salt to adjust its pH to 6.8. While the resulting solution was maintained at cold temperature, 0.2, 0.1, 0.02 or 0.01 mL of aqueous solution of glyoxal (87.2 mM) was added and vigorously homogenised. Transparent gels were formed at 37°C more or less rapidly depending on the glyoxal concentration.

**Table 3**

| **Homogeneous Chitosan Gel Cross-Linked with Glyoxal** | |
|---|---|
| **Glyoxal (mM)** | **Gelling Time at 37°C (min)** |
| 1.744 | immediate |
| 0.872 | immediate |
| 0.262 | 20 |
| 0.174 | 30 |
| 0.087 | 90 |

Homogeneous Chitosan Gels Cross-Linked with Polyethylene Glycol Diglycidyl Ether were prepared as delayed self-gelling systems: the experiment was performed as for Glyoxal, except that Glyoxal solution was replaced by polyethylene glycol diglycidyl ether.

**Table 4**

| **Homogeneous Chitosan Gel Cross-Linked with Polyethylene Glycol Diglycidyl Ether** | |
|---|---|
| **PEGDGly (mM)** | **Gelling Time at 37°C (h)** |
| 37.0 | 6 |
| 7.40 | 10 |
| 3.70 | 14 |
| 1.85 | 20 |
| 0.37 | No gelation |

### EXAMPLE III

### Preparation of rapid in situ gelling composition by grafting mPEG on chitosan in mild aqueous solution for in vivo administration

This example relates to aqueous compositions containing chitosan and mPEG that rapidly undergo gelation via the formation of covalent and non-covalent linkages between both polymers. The methoxy PEG-succinoyl-N-hydroxysuccinimide ester (mPEG-suc-NHS), and methoxy PEG-carboxymethyl-NHS (mPEG-cm-NHS) were reacted with chitosan under homogeneous conditions in mild aqueous solution to produce hydrogel formulations.

The hydrogel formulations were prepared by dissolving 200 mg of chitosan, (with medium viscosity and a degree of deacetylation of 90%) in 9 mL of HCl solution (0.1 M). The resulting solution was neutralized by adding 600 mg of β-GP dissolved in 1 mL of distilled water. The β-GP buffering solution was carefully added at low temperature (5°C) to obtain a clear and homogeneous liquid solution. The measured pH value of the final solution was 6.94. To the neutralized chitosan solution, 210 mg of mPEG-suc-NHS (M = 5197,17 g/mol) dissolved in 10 mL of water was added drop wise at room temperature. A transparent and homogeneous mPEG-grafted-chitosan gel was quickly obtained. No precipitate or aggregate was formed during or after the addition. To evidence the gel formation, rheological tests were performed. The gelling times of mPEG-grafted-chitosan at R.T. as a function of the mPEG-suc-NHS concentrations are summarized in Table 5.

**Table 5**

| **Gelling time at R.T. as a function of the mPEG-suc-NHS concentration** | | |
|---|---|---|
| **mPEG-suc-NHS (mg)** | **Molar ratio x 100 mPEG-suc-NHS/NH₂** | **Gelling Time at R.T. (min)** |
| 210 | 3.71 | 1 |
| 136 | 2.40 | 3 |
| 75 | 1.32 | 6 |
| 50 | 0.88 | 15 |
| 31 | 0.55 | 35 |
| 20 | 0.35 | 90 |

In a similar experiment, replacement of mPEG-suc-NHS by mPEG-cm-NHS led to similar results. Similar results were also obtained when the pH of chitosan solution has been adjusted, to around 6.9, by adding 150 mg of bis-tris (instead of β-GP) dissolved in 1 mL of water. We found that the gelling time also depends on the degree of deacetylation (DDA) and the pH, and that no gelation occurred if the pH value is below 6. Without the pH adjustment in the range 6.4 to 7.2, the grafting of mPEG on chitosan cannot occur and therefore the gelation cannot take place.

### EXAMPLE IV

### Preparation and Injection in situ of self-gelling Chitosan-mPEG formulation

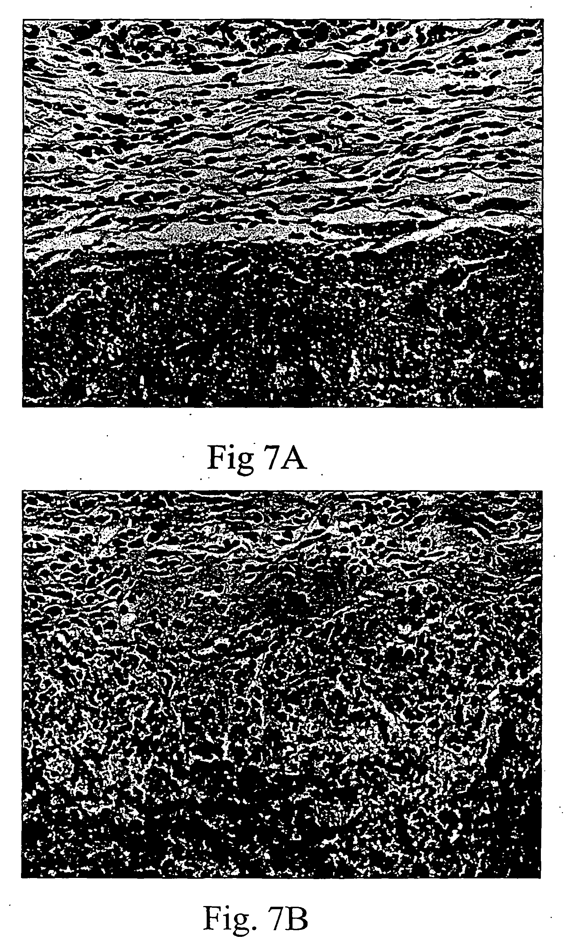

A Chitosan-mPEG aqueous solution was prepared by mixing a chitosan aqueous solution (pH = 6.6) and a methoxy-PEG-succinimide (mPEH-NHS). After 12 minutes of mixing, the chitosan-mPEG-NHS aqueous formulation was injected subcutaneously into Sprague-Dawley rats, using a hypodermic syringe and a gauge 18 needle. Rats were sacrificed periodically from 3 days and up to 56 days. The chitosan-mPEG-NHS gel materials were collected, fixed in appropriate buffer and histopathological analyzed. All animal procedures followed the rules of the Canadian Committee for Animal Care. Figs. 7A and 7B show the histological slides of Chitosan-mPEG-NHS (Fig. 7A) and Chitosan (Fig. 7B) gel materials at 21 days implantation. Staining was Saffranin-O/Fast Green (magnification x40).

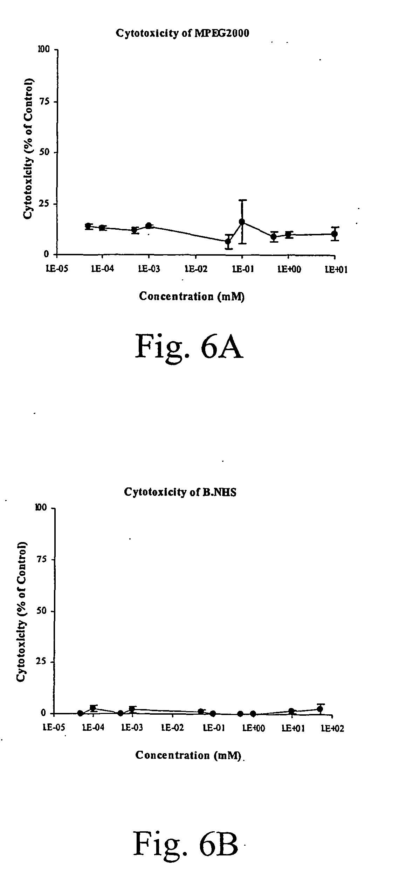

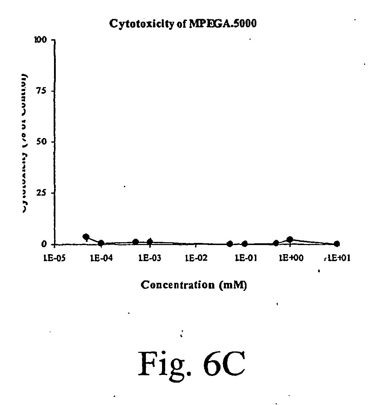

Methoxy-poly(ethylene glycol) compounds were also evaluated *in vitro* in terms of cytotoxicity, by direct culture of adherent murine macrophage J774 cells in presence of various concentrations of mPEG compounds, namely mPEG-N-hydroxysuccinimide (mPEG-NHS) and mPEG-carboxylic acid (mPEG-CA). Cells were incubated for 6 hours with increasing concentrations of mPEG compounds, in RPMI supplemented with 1% FBS. Cytotoxicity was assessed using a lactate dehydrogenase (LDH) release assay. In Figs. 6A to 6C, the Control is Triton-treated cells and represents maximum LDH activity. Data represents means ± st. dev., N=3 or 4.

*In vitro* results showed that cytotoxicity tests with mPEG compounds display minimal to no cytotoxicity compared to controls. *In vivo* results demonstrated a) the chitosan-mPEG-NHS gels form uniformly and homogeneously *in situ,* and b) chitosan-mPEG-NHS materials display relatively high level of biocompatibility.

### EXAMPLE V

### Injection into Cow tail and Beagle inter-vertebral disk nucleus

The coloured material has been injected into the disc nucleus of the spines of two Beagle dogs as well as in the disc nucleus of the spine of Cow tails. For beagles, all lumbar discs, from thoracic 13/lumbar 1 (T13-L1) to lumbar 4/lumbar 5 (L4-L5) were injected in this fashion.

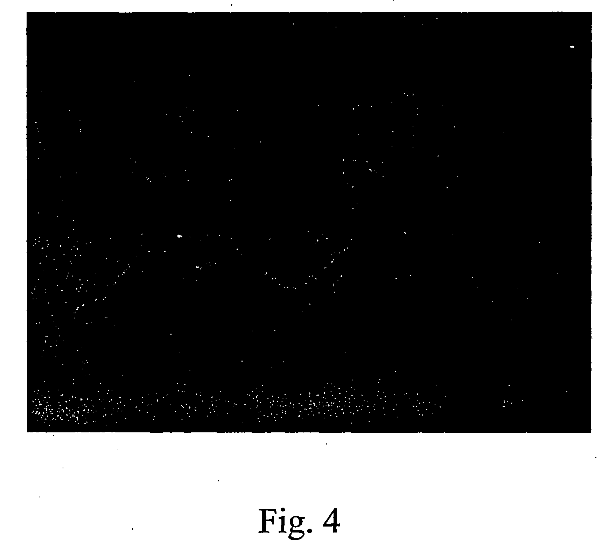

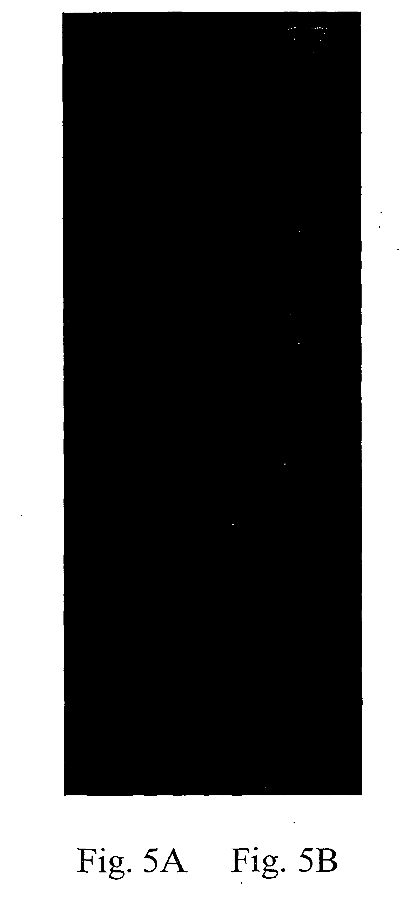

On Beagles, lateral X-rays were taken before and after the injections. Those images were then digitised, and the labels on the images were removed to blind the analysis. The thickness of each disc on the images were then measured by Image analysis, by averaging three independent measurments. On Beagle disc, the results showed that the injection increases on average the disc thickness by 0.25±0.02mm, on average (Figs. 4, 5A and 5B). The spines were dissected, and the discs transected. As shown by examples with coloured gel, the product enters the nucleus pulposus and mixes with the nucleus, without leaking in the annulus. In Fig. 4, it can be seen that the gel remains circumscribed within the nucleus pulposus, and mixes with its substance.

A series of biomechanical tests were performed on the cadaveric Cow spines. Vertebral segments, uninjected or injected with the gel were cast in resin and fitted in a biomechanical testing system. The segments were maintained moist and submitted to a series of compressions. The stress-strain relationships of the assemblies were measured during a 10,000 cycles at 1 Hertz, and 5% deformation. The results demonstrated that the injection of gel rigidifies the segment and increases its elastic modulus by 30±4% at the onset of the cycling deformations. This difference remains essentially equal throughout the tests, decreasing to 25±4% at the end of the 10,000 cycles, thus showing the persistence of the gel action.

## Claims

1. The use of a nucleus pulposus formulation comprising:
a) 0.1 to 5.0% by weight of a water-soluble polymer selected from the group consisting of cellulosic, polysaccharide and polypeptidic, or a mixture thereof, and
b) 1.0 to 20% by weight of a water-soluble salt selected from the group consisting of phosphate, glycerol-phosphate, glucose-phosphate, fructose phosphate, carbonate, sulfate, sulfonate and a salt of polyol or sugar selected from the group consisting of mono-phosphate dibasic salt, mono-sulfate salt and a mono-carboxylic acid salt;
wherein said formulation has a pH ranging from 6.5 to 7.4, and turns into a gel within a temperature range from 20 to 70°C, said gel having a physiologically acceptable consistency for increasing the thickness of the disc, providing a mechanical support once injected in the disc, in the manufacture of a medicament for restoring a damaged or degenerated intervertebral disk.

2. The use of a nucleus pulposus formulation according to claim 1, wherein:
a) said water-soluble polymer comprises 0.1% to 5% by weight of a water-soluble polymer selected from the group consisting of chitosan and collagen, or a mixture thereof, and
b) said water-soluble salt comprises 1.0 to 20% by weight of a water soluble salt selected from the group consisting of phosphate, carbonate, sulfate, sulfonate and a salt of polyol or sugar selected from the group consisting of mono-phosphate dibasic salt, mono-sulfate salt, and a mono-carboxylic acid salt;
wherein said formulation has a pH ranging from 6.5 to 7.4, and turns into a gel within a temperature range from 20 to 70°C, said gel having a physiologically acceptable consistency for increasing the thickness of the disc, providing a mechanical support once injected in the disc, in the manufacture of a medicament for restoring a damaged or degenerated invertebral disc.

3. The use of a nucleus pulposus formulation comprising:
a) 0.1 to 5.0% by weight of chitosan or collagen or a derivative thereof, or a mixture thereof ; and
b)
i) 1.0 to 20% by weight of a salt of polyol or sugar selected from the group consisting of mono-phosphate dibasic salt, mono sulfate salt and a mono-carboxylic acid salt of polyol or sugar; or ii) 1.0 to 20% by weight of a salt selected from the group consisting of phosphate, carbonate, sulfate, and sulfonate, or the like; and
c) 0.01 to 10% by weight of a water-soluble chemically reactive organic compound;
wherein said formulation has a pH ranging from 6.5 to 7.4, and turns into a gel within a temperature range from 4 to70 C, said gel having a physiologically acceptable consistency for increasing the thickness of the disc, providing a mechanical support once injected in the disc, in the manufacture of a medicament for restoring of a damaged or degraded invertebral disc.

4. The use of claim 3, wherein said water-soluble chemically reactive organic compound is a functionalized poly(ethylene glycol).

5. The use of claim 4, wherein said wstar-soluble chemically reactive organic compound is a monofunctional methoxy-poly(ethylene glycol).

6. The use of claim 4, wherein said water-soluble chemically reactive organic compound is a multifunctional poly(ethylene glycol).

7. The use of claim 3, wherein said water-soluble chemically reactive organic compound is selected from the group consisting of aldehyde, anhydride acid, azide, azolide, carboimide, carboxylic acid, epoxide, esters, glycidyl ether, halide, imidazole, imidate, succinimide, succinimidyl ester, acrylate and methacrylate, or a mixture thereof.

8. The use of claim 2, wherein the nucleus pulposus formulation comprises 0.1 to 3.0% of a chitosan, and 1.0 to 10% of a water-soluble phosphate salt and which turns into gel within a temperature range from 20 to 40°C.

9. The use of claim 8, wherein the nucleus pulposus formulation comprises 0.01 to 5% by weight of a water-soluble chemically reactive organic compound.

10. The use of any one of claims 1 to 9, wherein said nucleus pulposus formulation further comprises a nonsoluble particulate material.

11. The use of claim 10, wherein said nonsoluble particulate material is a biodegradable organic particulate material.

12. The use of claim 11, wherein said biodegradable organic particulate material is made of an absorbable polymer.

13. The use of claim 12, wherein said absorbable polymer is selected from the group consisting of poly(lactic acid), poly(glycolic acid), poly(lactic-co-glycolic), poly(lactones), poly(orthoesters), poly(anhydrides) (anhydrides), and poly(carbonates).

14. The use of claim 13, wherein said biodegradable organic particulate material is made of gelatin, collagen.

15. The use of claim 10, wherein said nonsoluble particulate material is an inorganic or mineral particulate material.

16. The use of claim 15, wherein said inorganic or mineral particulate material is selected from the group consisting of bioglass, calcium phosphate, and calcium carbonate.

17. The use of any one of claims 1 to 9, wherein said nucleus pulposus formulation further comprises cells.

18. The use of claim 17, wherein said cells are autologous.

19. The use of claim 17, wherein said cells are modified.

20. The use of claim 17, wherein said cells are stem cells or chondrocytes.

21. The use of any one of claims 1 to 9, wherein said nucleus pulposus formulation further comprises a bioactive or pharmaceutical agent.

22. The use of claim 21, wherein said bioactive or pharmaceutical agent is a cell stimulant, a cell preservative, or a cell differentiation factor.

23. The use of claim 21, wherein said bioactive or pharmaceutical agent is a cytokine or a growth factor.

24. The use of claim 21, wherein said bioactive or pharmaceutical agent is an antipain or anti-inflammation drug.

25. The use of any one of claims 1 to 24, wherein said nucleus pulposus formulation forms a viscous, gel, pasty or solid material *in situ.*

26. The use of any one of claims 1 to 9, wherein said nucleus pulposus formulation has a viscosity above 10 mPa.s at the time of administration.

## Patentansprüche

1. Verwendung einer Gallertkernzubereitung, die Folgendes umfasst:
a) 0,1 bis 5,0 Gew.-% eines wasserlöslichen Polymers, das aus der Gruppe ausgewählt ist, die aus Cellulose-, Polysaccharid- und Polypeptid-Polymeren oder einem Gemisch davon besteht; und
b) 1,0 bis 20 Gew.-% eines wasserlöslichen Salzes, das aus der Gruppe ausgewählt ist, die aus Phosphat, Glycerinphosphat, Glucosephosphat, Fructosephosphat, Carbonat, Sulfat, Sulfonat und einem Salz eines Polyols oder Zuckers besteht, das aus der Gruppe ausgewählt ist, die aus dibasischem Monophosphatsalz, Monosulfatsalz und einem Monocarbonsäuresalz besteht;
wobei die Zubereitung einen pH-Wert im Bereich von 6,5 bis 7,4 hat und innerhalb eines Temperaturbereichs von 20 bis 70 °C zu einem Gel wird, wobei das Gel eine physiologisch annehmbare Konsistenz zur Erhöhung der Dicke der Bandscheibe hat und für einen mechanischen Halt sorgt, sobald es in die Bandscheibe injiziert ist;
bei der Herstellung eines Medikaments zur Wiederherstellung einer geschädigten oder degenerierten Bandscheibe.

2. Verwendung einer Gallertkernzubereitung gemäß Anspruch 1, wobei:
a) das wasserlösliche Polymer 0,1 bis 5 Gew.-% eines wasserlöslichen Polymers umfasst, das aus der Gruppe ausgewählt ist, die aus Chitosan und Collagen oder einem Gemisch davon besteht; und
b) das wasserlösliche Salz 1,0 bis 20 Gew.-% eines wasserlöslichen Salzes umfasst, das aus der Gruppe ausgewählt ist, die aus Phosphat, Carbonat, Sulfat, Sulfonat und einem Salz eines Polyols oder Zuckers besteht, das aus der Gruppe ausgewählt ist, die aus dibasischem Monophosphatsalz, Monosulfatsalz und einem Monocarbonsäuresalz besteht;
wobei die Zubereitung einen pH-Wert im Bereich von 6,5 bis 7,4 hat und innerhalb eines Temperaturbereichs von 20 bis 70 °C zu einem Gel wird, wobei das Gel eine physiologisch annehmbare Konsistenz zur Erhöhung der Dicke der Bandscheibe hat und für einen mechanischen Halt sorgt, sobald es in die Bandscheibe injiziert ist; bei der Herstellung eines Medikaments zur Wiederherstellung einer geschädigten oder degenerierten Bandscheibe.

3. Verwendung einer Gallertkernzubereitung, die Folgendes umfasst:
a) 0,1 bis 5,0 Gew.-% Chitosan oder Collagen oder eines Derivats davon oder eines Gemischs davon; und
b)
i) 1,0 bis 20 Gew.-% eines Salzes eines Polyols oder Zuckers, das aus der Gruppe ausgewählt ist, die aus dibasischem Monophosphatsalz, Monosulfatsalz und einem Monocarbonsäuresalz von Polyol oder Zucker besteht; oder
ii) 1,0 bis 20 Gew.-% eines Salzes, das aus der Gruppe ausgewählt ist, die aus Phosphat, Carbonat, Sulfat und Sulfonat oder dergleichen besteht; und
c) 0,01 bis 10 Gew.-% einer wasserlöslichen chemisch reaktiven organischen Verbindung;
wobei die Zubereitung einen pH-Wert im Bereich von 6,5 bis 7,4 hat und innerhalb eines Temperaturbereichs von 4 bis 70 °C zu einem Gel wird, wobei das Gel eine physiologisch annehmbare Konsistenz zur Erhöhung der Dicke der Bandscheibe hat und für einen mechanischen Halt sorgt, sobald es in die Bandscheibe injiziert ist;
bei der Herstellung eines Medikaments zur Wiederherstellung einer geschädigten oder degenerierten Bandscheibe.

4. Verwendung gemäß Anspruch 3, wobei die wasserlösliche chemisch reaktive organische Verbindung ein funktionalisiertes Polyethylenglycol ist.

5. Verwendung gemäß Anspruch 4, wobei die wasserlösliche chemisch reaktive organische Verbindung ein monofunktionelles Methoxypolyethylenglycol ist.

6. Verwendung gemäß Anspruch 4, wobei die wasserlösliche chemisch reaktive organische Verbindung ein multifunktionelles Polyethylenglycol ist.

7. Verwendung gemäß Anspruch 3, wobei die wasserlösliche chemisch reaktive organische Verbindung aus der Gruppe ausgewählt ist, die aus Aldehyd, Anhydrid, Säure, Azid, Azolid, Carboimid, Carbonsäure, Epoxid, Estern, Glycidylether, Halogenid, Imidazol, Imidat, Succinimid, Succinimidylester, Acrylat und Methacrylat oder einem Gemisch davon besteht.

8. Verwendung gemäß Anspruch 2, wobei die Gallertkernzubereitung 0,1 bis 3,0% eines Chitosans und 1,0 bis 10% eines wasserlöslichen Phosphatsalzes umfasst und innerhalb eines Temperaturbereichs von 20 bis 40 °C zu einem Gel wird.

9. Verwendung gemäß Anspruch 8, wobei die Gallertkernzubereitung 0,01 bis 5 Gew.-% einer wasserlöslichen chemisch reaktiven organischen Verbindung umfasst.

10. Verwendung gemäß einem der Ansprüche 1 bis 9, wobei die Gallertkernzubereitung weiterhin ein unlösliches teilchenförmiges Material umfasst.

11. Verwendung gemäß Anspruch 10, wobei das unlösliche teilchenförmige Material ein biologisch abbaubares organisches teilchenförmiges Material ist.

12. Verwendung gemäß Anspruch 11, wobei das biologisch abbaubare organische teilchenförmige Material aus einem resorbierbaren Polymer besteht.

13. Verwendung gemäß Anspruch 12, wobei das resorbierbare Polymer aus der Gruppe ausgewählt ist, die aus Polymilchsäure, Polyglycolsäure, Poly(milchsäure-co-glycolsäure), Polylactonen, Polyorthoestern, Polyanhydriden und Polycarbonaten besteht.

14. Verwendung gemäß Anspruch 13, wobei das biologisch abbaubare organische teilchenförmige Material aus Gelatine, Collagen besteht.

15. Verwendung gemäß Anspruch 10, wobei das unlösliche teilchenförmige Material ein anorganisches oder mineralisches teilchenförmiges Material ist.

16. Verwendung gemäß Anspruch 15, wobei das anorganische oder mineralische teilchenförmige Material aus der Gruppe ausgewählt ist, die aus Bioglas, Calciumphosphat und Calciumcarbonat besteht.

17. Verwendung gemäß einem der Ansprüche 1 bis 9, wobei die Gallertkernzubereitung weiterhin Zellen umfasst.

18. Verwendung gemäß Anspruch 17, wobei die Zellen autolog sind.

19. Verwendung gemäß Anspruch 17, wobei die Zellen modifiziert sind.

20. Verwendung gemäß Anspruch 17, wobei die Zellen Stammzellen oder Chondrocyten sind.

21. Verwendung gemäß einem der Ansprüche 1 bis 9, wobei die Gallertkernzubereitung weiterhin ein bioaktives oder pharmazeutisches Mittel umfasst.

22. Verwendung gemäß Anspruch 21, wobei das bioaktive oder pharmazeutische Mittel ein Zellstimulans, Zellkonservierungsstoff oder Zelldifferenzierungsfaktor ist.

23. Verwendung gemäß Anspruch 21, wobei das bioaktive oder pharmazeutische Mittel ein Cytokin oder Wachstumsfaktor ist.

24. Verwendung gemäß Anspruch 21, wobei das bioaktive oder pharmazeutische Mittel ein Schmerzmittel oder entzündungshemmendes Mittel ist.

25. Verwendung gemäß einem der Ansprüche 1 bis 24, wobei die Gallertkernzubereitung in situ ein viskoses, gelartiges, pastöses oder festes Material bildet.

26. Verwendung gemäß einem der Ansprüche 1 bis 9, wobei die Gallertkernzubereitung zum Zeitpunkt der Verabreichung eine Viskosität von über 10 mPa·s hat.

## Revendications

1. L'usage d'une préparation de nucleus pulposus comprenant :
a) 0,1 à 5,0% en poids d'un polymère soluble dans l'eau choisi parmi le groupe constitué par un polymère cellulosique, un polysaccharide et un polymère polypeptidique, ou un mélange de ces derniers, et
b) 1,0 à 20% en poids d'un sel soluble dans l'eau choisi parmi le groupe constitué par un phosphate, un phosphate de glycérol, un phosphate de glucose, un phosphate, carbonate, sulfate, sulfonate de fructose et un sel d'un polyol ou un sucre choisi parmi le groupe constitué par un sel de mono-phosphate dibasique, un sel d'un mono-sulfate et un sel d'un acide monocarboxylique;
**caractérisé en ce que** ladite préparation possède un pH se situant entre 6,5 et 7,4, et se transforme en un gel dans une gamme de température se situant entre 20 et 70° C, ledit gel ayant une consistance physiologiquement acceptable permettant d'augmenter l'épaisseur du disque, assurant un support mécanique une fois qu'il est injecté dans le disque, dans la fabrication d'un médicament pour la restauration d'un disque intervertébral endommagé ou dégénéré.

2. L'usage d'une préparation de nucleus pulposus selon la revendication 1, dans lequel :
a) ledit polymère soluble dans l'eau comprend 0,1% à 5% en poids d'un polymère soluble dans l'eau choisi parmi le groupe constitué d'un chitosane et un collagène, ou un mélange de ces derniers, et
b) ledit sel soluble dans l'eau comprend 1,0 à 20% en poids d'un sel soluble dans l'eau choisi parmi le groupe constitué par un phosphate, carbonate, sulfate, sulfonate et un sel d'un polyol ou d'un sucre choisi parmi le groupe constitué par un sel de mono-phosphate dibasique, d'un sel de mono-sulfate, et un sel d'acide monocarboxylique;
**caractérisé en ce que** ladite préparation possède un pH se situant entre 6,5 et 7,4, et se transforme en un gel dans une gamme de température variant entre 20 et 70° C, ledit gel possédant une consistance physiologiquement acceptable permettant d'augmenter l'épaisseur du disque, assurant un support mécanique une fois qu'il est injecté dans le disque, dans la fabrication d'un médicament pour la restauration d'un disque intervertébral endommagé ou dégénéré.

3. L'usage d'une préparation de nucleus pulposus comprenant :
a) 0,1 à 5,0% en poids de chitosane ou de collagène ou un dérivé de ces derniers; et
b)
i) 1,0 à 20% en poids d'un sel d'un polyol ou d'un sucre choisi parmi le groupe constitué par un sel de mono-phosphate dibasique, un sel de mono-sulfate et un sel d'acide monocarboxylique d'un polyol ou d'un sucre; ou
ii) 1,0 à 20% en poids d'un sel choisi parmi le groupe constitué par un phosphate, un carbonate, un sulfate, et un sulfonate, ou autre de même nature; et
c) 0,01 à 10% en poids d'un composé organique soluble dans l' eau et chimiquement réactif;
**caractérisé en ce que** ladite préparation possède un pH se situant entre 6,5 et 7,4, et se transforme en un gel dans une gamme de température allant de 4 à 70° C, ledit gel ayant une consistance physiologiquement acceptable, capable de faire augmenter l'épaisseur du disque, assurant un support mécanique une fois injecté dans le disque, dans la fabrication d'un médicament pour la restauration d'un disque invertébral endommagé ou dégradé.

4. L'usage de la revendication 3, dans lequel ledit composé organique chimiquement réactif soluble dans l'eau est un glycol polyéthylénique fonctionnalisé.

5. L'usage de la revendication 4, dans lequel ledit composé organique chimiquement réactif soluble dans l'eau est un méthoxy-glycol polyéthylénique monofonctionnel.

6. L'usage de la revendication 4, dans lequel ledit composé organique chimiquement réactif soluble dans l'eau est un glycol polyéthylénique multifonctionnel.

7. L'usage de la revendication 3, dans lequel ledit composé organique chimiquement réactif soluble dans l'eau est choisi parmi le groupe constitué par un aldéhyde, un anhydride d'acide, un azide, un azolide, un carboimide, un acide carboxylique, un époxyde, les esters, un éther glycidylique, un halogénure, un imidazole, un imidate, un succinimide, un ester succinimidylique, un acrylate et un méthacrylate, ou les mélanges de ces derniers.

8. L'usage de la revendication 2, dans lequel la préparation de nucleus pulposus comprend 0,1 à 3,0% d'un chitosane, et 1,0 à 10% d'un sel de phosphate soluble dans l'eau et qui se transforme en gel dans une gamme de température se situant entre 20 et 40° C.

9. L'usage de la revendication 8, dans lequel la préparation de nucleus pulposus comprend 0,01 à 5% en poids d'un composé organique chimiquement réactif soluble dans l'eau.

10. L'usage de l'une quelconque des revendications 1 à 9, dans lequel ladite préparation de nucleus pulposus comprend en outre un matériau particulaire non soluble.

11. L'usage de la revendication 10, dans lequel ledit matériau particulaire est un matériau particulaire organique biodégradable.

12. L'usage de la revendication 11, dans lequel ledit matériau particulaire organique biodégradable est constitué d'un polymère résorbable.

13. L'usage de la revendication 12, dans lequel ledit polymère résorbable est choisi parmi l'acide poly(lactique), l'acide poly(glycolique), l'acide poly(lactique-co-glycolique), les poly(lactones), les poly(orthoesters), les poly(anhydrides)(anhydrides), et les poly(carbonates).

14. L'usage de la revendication 13, dans lequel ledit matériau particulaire organique biodégradable est constitué de gélatine, de collagène.

15. L'usage de la revendication 10, dans lequel ledit matériau particulaire non soluble est un matériau particulaire inorganique ou minéral.

16. L'usage de la revendication 15, dans lequel ledit matériau particulaire inorganique ou minéral est choisi parmi le groupe constitué de bioverre, de phosphate de calcium, et de carbonate de calcium.

17. L'usage de l'une quelconque des revendications 1 à 9, dans lequel ladite préparation de nucleus pulposus comprend en outre des cellules.

18. L'usage de la revendication 17, dans lequel lesdites cellules sont autologues.

19. L'usage de la revendication 17, dans lequel lesdites cellules sont modifiées.

20. L'usage de la revendication 17, dans lequel lesdites cellules sont des cellules souches ou des chondrocytes.

21. L'usage de l'une quelconque des revendications 1 à 9, dans lequel ladite préparation de nucleus pulposus comprend en outre un agent bioactif ou pharmaceutique.

22. L'usage de la revendication 21, dans lequel ledit agent bioactif ou pharmaceutique est un stimulant de cellules, un préservateur de cellules, ou un facteur de différentiation cellulaire.

23. L'usage de la revendication 21, dans lequel ledit agent bioactif ou pharmaceutique est une cytokine ou un facteur de croissance.

24. L'usage de la revendication 21, dans lequel ledit agent bioactif ou pharmaceutique est un médicament anti-douleur ou anti-inflammatoire.

25. L'usage de l'une quelconque des revendications 1 à 24, dans lequel ladite préparation de nucleus pulposus forme un matériau visqueux, un matériau sous forme de gel, de pâte ou solide *in situ.*

26. L'usage de l'une quelconque des revendications 1 à 9, dans lequel ladite préparation de nucleus pulposus possède une viscosité supérieure à 10 mPa lors de l'administration.
